Europäisches Patentamt

European Patent Office

Office européen des brevets

(11) Publication number: **0 242 216**
**A1**

# EUROPEAN PATENT APPLICATION

(21) Application number: **87303363.3**

(22) Date of filing: **15.04.87**

(51) Int. Cl.⁴: **C 12 N 15/00**
**C 12 N 7/00, A 61 K 39/12**

(30) Priority: **16.04.86 US 852531**

(43) Date of publication of application:
**21.10.87 Bulletin 87/43**

(84) Designated Contracting States:
**AT BE CH DE FR GB IT LI LU NL SE**

(71) Applicant: **THE UNITED STATES OF AMERICA**
**represented by The Secretary The United States**
**Department of Commerce**
**5285 Port Royal Road**
**Springfield Virginia 22161 (US)**

(72) Inventor: **Fisher, Amanda Gay**
**5514 Johnson Avenue**
**Bethesda Maryland 20817 (US)**

**Wong-Staal, Florrie**
**3 Lynn Manor Court**
**Potomac Maryland 20854 (US)**

**Gallo, Robert Charles M.D.**
**8513 Thornden Terrace**
**Bethesda Maryland 20834 (US)**

**Ratner, Lee**
**7336 Northmore Drive**
**St. Louis Missouri 63105 (US)**

(74) Representative: **Jump, Timothy John Simon et al**
**F.J. Cleveland and Company 40-43 Chancery Lane**
**London WC2A 1JQ (GB)**

(54) **Non-cytopathic clone of Human T-Cell Lymphotropic Virus type III.**

(57) The X10-1 variant of a molecular clone of Human T-cell Lymphotropic Virus Type III is infectious and noncytopathic in vitro. This variant is a deletion mutant wherein the last 14 base pairs of the envelope gene, 4 base pairs intervening between the env gene and the 3'orf gene, and the first 182 base pairs of the 3'orf gene have been deleted.

FIG.1

EP 0 242 216 A1

**Description**

## NON-CYTOPATHIC CLONE OF HUMAN T-CELL LYMPHOTROPIC VIRUS TYPE III

The first direct evidence that a product of the Human T-Cell Lymphotropic Virus Type III (HTLV-III) genome mediates cell-killing was shown by the capacity of a molecular clone of HTLV-III to generate infectiuous virions and cytopathic effects on normal T lymphocytes in vitro. This evidence was published in Fisher et al, Nature, Vol. 316, pp. 262-265 (1985). The present invention comprises the first molecular clone of HTLV-III which generates infectious virions, which are not cytopathic to normal T lymphocytes. Such an invention reveals a new area for the development of a safe vaccine in the treatment of Acquired Immune Deficiency Syndrome (AIDS).

Due to the noncytopathogenicity of the present invention, both whole virus and segments of the virus may be safely used as immunogens according to the present invention. To date, vaccine development has concentrated on cytopathic HTLV-III clones and their derivatives. It is believed that these cytopathic clones are potentially harmful to vaccine recipients. The molecular clone of the present invention is noncytopathic, and therefore eliminates the potential hazards of all cytopathic clones in the development of a safe and effective vaccine.

Human T-cell Lymphotropic Virus Type III is a retrovirus causally linked to Acquired Immune Deficiency Syndrome. In conjunction with an immortalizing T-cell line H9, diagnostic procedures and test kits have been developed (U.S. Patent No. 4,520,113). The immunosuppressive effects of HTLV-III in vivo are largely paralled by its ability to selectively infect and kill OKT4 helper/inducer cells in vitro. DNA transfection studies have shown that this cytopathic effect is intrinsic to the HTLV-III genome and have also provided a means to dissect the viral determinants of pathogenicity.

The HTLV-III genome is unusually complex for a retrovirus, possessing in addition to the replicative genes (gag, pol, and env) at least three extra genes (sor, tat, and 3'orf). Of these, the transactivator gene of HTLV-III (tat-III) has been determined and shown to be critical to virus replication. The sor and 3'orf genes, originally identified as open reading frames, have been shown to encode proteins which are immunogenic in vivo, but the functions of these gene products are, as yet, unknown.

Since immunosuppression in AIDS patients is attributable to HTLV-III infecting and killing helper/inducer lymphocytes, identification of the viral determinant(s) of cytopathogenicity is critical in the development of safe therapeutic and preventative treatments for AIDS. The present invention shows that the cytopathic elements of HTLV-III are localized to a 93 base pair segment spanning the 3'orf and env genes.

The present invention is directed to the production of X10-1, a deletion variant of a Human T-cell Lymphotropic Virus Type III molecular clone, which is infectious and non-cytopathic in vitro.

Description of the Figures

Figure 1 illustrates the nucleotide mapping of the present invention.

Figure 2 illustrates infection of H9 cells by HTLV-III variants.

Figure 3 illustrates Example 3 of the specification.

Figure 4 illustrates the comparative cytopathic effects of HTLV-III variants on ATH8 cells.

A molecular clone of this invention has been deposited in the American Type Culture Collection, 12301 Parklawn Drive, Rockville, Maryland 20852-1776, U.S.A., under ATCC No. 67083, on April 16, 1986, and will be maintained for a term of thirty (30) years or five (5) years after the last request for such deposit or for the effective life of the patent, whichever is longest. The deposit will be replaced if the culture mutates or becomes nonviable during the term of the deposit.

The present invention is directed to a molecular clone of Human T-cell Lymphotropic Virus Type III (HTLV-III), which produces an infectious virus which is noncytopathic toward human T cells, preferably such a clone which has the characteristics of the molecular clone deposited in the American Type Culture Collection under Accession #67083. The subject molecular clone is biologically active. It includes a deletion in the envelope gene/3' orf gene region. The deletion includes the last five codons of the envelope gene. Preferably, it also includes 25 codons, more preferably the first 60 codons, of the 3' orf gene. The deletion preferably comprises the last 14 base pairs of the envelope gene and the first 182 base pairs of the 3' orf gene, and preferably also the 4 base pair intervening sequence between the envelope gene and the 3' orf gene. The invention is further directed to a process for producing such a molecular clone, characterized by deleting a segment corresponding to the aforesaid deletion from Human T-Cell Lymphotropic Virus Type III.

The starting material used in the construction of the HTLV-III variant of the present invention is a molecular clone of Human T-cell Lymphotropic Virus Type III (HTLV-III), pHXB2D, and contains a full length, biologically active HTLV-III genome (9.6kb) flanked by long terminal repeat (LTR) sequences. While pHXB2D is the preferred molecular clone of this invention, other biologically active molecular clones may be substituted in the practice of this invention. Also, isolated segments of clones may be substituted in the practice of this invention. As is described in more detail in Fisher et al, Nature, Vol. 316, pp. 262-265 (1985), this molecular clone is constructed by inserting a 12.7kb Xba I fragment (derived from lambda HXB-2D, a phage clone containing about a 10kb segment of HTLV-III proviral sequences) into the Xba I site in the polylinker of plasmid pSP62. The genome contained in pHXB2D contains a single XhoI and BamHI site in the 3' orf and env genes respectively (see Shaw et al, Science, Vol. 226, pp. 1165-1171 (1984)).

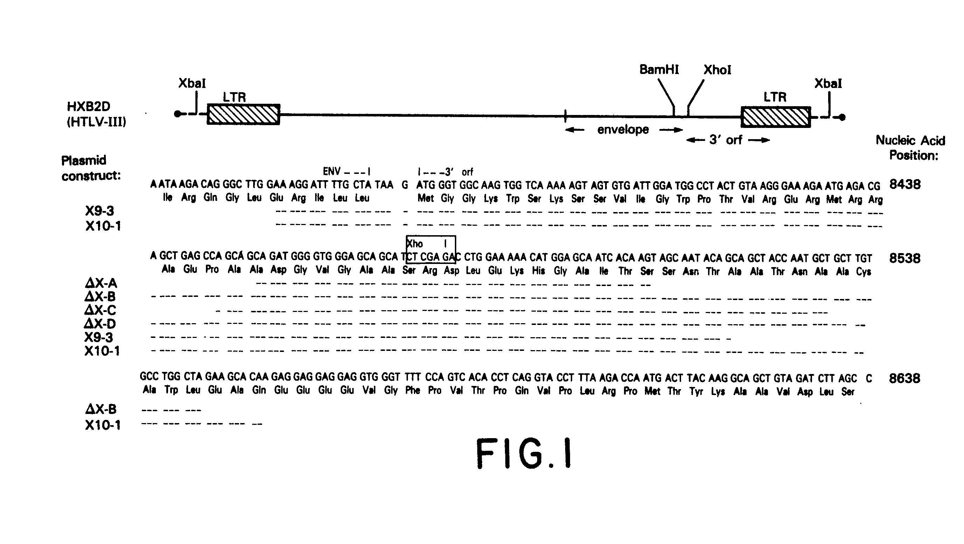

The proviral fragment of pHXB2D is inserted into pSP65gpt (a vector carrying the xanthine guanine phosphoribosyl transferase gene but devoid of HTLV-III sequences) in order to generate plasmid pHXB2gpt. The variants of the present invention (which contain deletions at the XhoI site in the viral genome) are produced by digesting pHXB2gpt to completion with XhoI, treating with Bal 31, blunt-ending using T4 DNA polymerase, and then self-ligating with T4 DNA ligase. The precise nature of the variant is analyzed by nucleotide sequencing. The position and extent of the deleted segments relative to the nucleotide and amino acid sequence of pHXB2gpt are shown in the middle of Figure 1 (shown as ---).

Plasmid clone X10-1, constructed by the above described process, is then introduced into H9 cells using the protoplast fusion technique of Yoakum et al, Science, Vol. 222, pp. 385-389 (1983). Briefly, $10^{10}$ plasmid bearing bacterial protoplasts are combined with 3 x $10^6$ H9 cells, treated for one minute with 48% polyethylene glycol, diluted, washed and returned to culture in RPMI 1640 medium containing 20% fetal calf serum and antibiotics. In the above-noted step of digesting pHXB2gpt to completion with XhoI, deletion variants of the biologically active molecular clone pHXB2D are formed. This process makes use of the unique XhoI restriction site in the 3' orf region of the viral element of pHXB2gpt. Using the Bal 31 digestion technique (Maniatis et al (eds.), Molecular Cloning: A Laboratory Manual (Cold Spring Harbor Laboratory, New York, 1982)), sequences deleted from clone X10-1 encompass the last 14 base pairs (the carboxy terminus) of the envelope gene, 4 base pairs intervening between the env and 3'orf genes, and up to the first 182 base pairs of the 3' orf gene. While the present invention includes deletions in the 3' orf of less than the first 182 base pairs, it has been determined that, at most, the last 14 base pairs of the envelope gene must be deleted in order to produce a non-cytopathic variant of HTLV-III. For example, a variant genome, comparable to X10-1, harbored an 85 base pair deletion exclusively in 3' orf; this variant produced a virus which was extremely cytopathic to human T cells. In the preferred embodiment, the deletion includes but is not limited to 14 base pairs of the 3' end of the env gene, the intervening sequences between the env and 3'orf genes (4 base pairs), plus 75-182 base pairs of the 3'orf gene.

Furthermore, the cytopathic effects of the HTLV-III clones known to practitioners in the art have been localized to starting with the 14 base pairs of the env gene and ending with the first 75 base pairs of the 3'orf gene (the total segment deleted is 93 base pairs long). A variant clone wherein this 93 base pairs segment has been deleted is also included within the scope of this invention. The capacity of the variant genome X10-1 to generate HTLV-III virions is determined by transfecting the X10-1 plasmid into the Human T Lymphoid Cell Line, H9. The production of cultures expressing HTLV-III gag (p17 and p24) and env (p41) proteins is shown in Figure 1.

As is shown in Example 5, X10-1 is infectious.

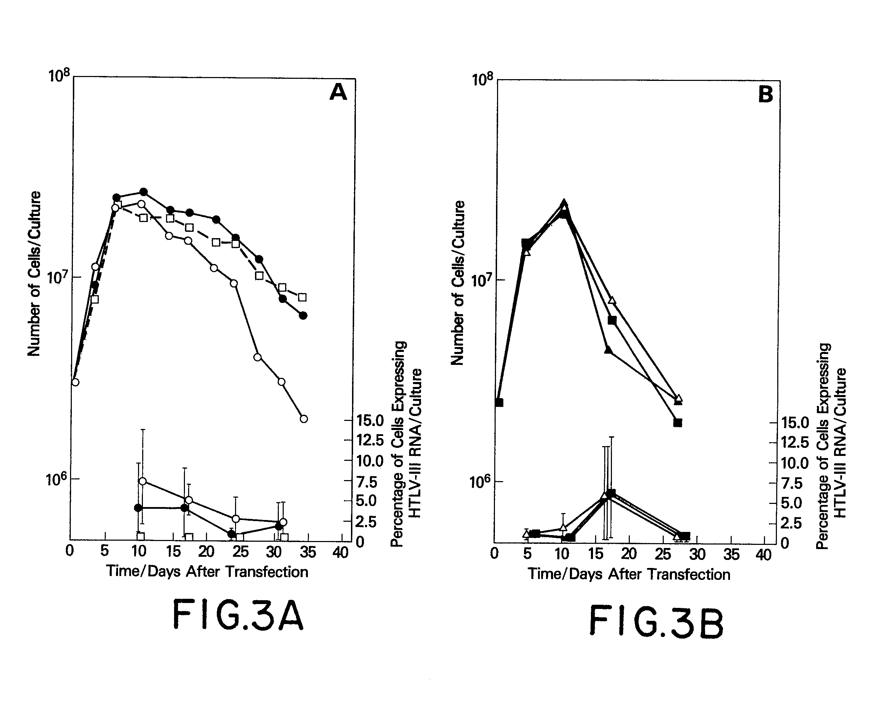

As is shown in Example 6 and Figures 3A and 3B, variant genome X10-1 is non-cytopathic.

In short, the present inventors have produced an HTLV-III variant genome (X10-1) in which the last 5 codons of the envelope gene through the first 60 codons of 3' orf gene are deleted; this variant is fully competent for virus expression, but is no longer capable of killing human T cells.

Examples

Example 1

Construction and replicative capacity of HTLV-III clones deleted in the 3'region of the virus. The molecular clone pHXB2D contains a full length, biologically active HTLV-III genome (9.6 kb) flanked by long terminal repeat (LTR) sequences (Figure 1, cross-hatched boxes), contained within the plasmid vector pSP62. This genome has been extensively characterized and contains a single XhoI and BamHI site in the 3' orf and env genes respectively. Plasmid pHXB2gpt, a derivative of this clone in which the proviral fragment of pHXB2D is inserted into pSP65gpt (a vector carrying the xanthine guanine phosphoribosyl transferase gene but devoid of HTLV-III sequences) was generated and used to construct variants bearing deletions at the XhoI site in the viral genome; briefly, pHXB2gpt was digested to completion with XhoI, treated with Bal 31, blunt ended using T4 DNA polymerase and then self ligated with T4 DNA ligase. The precise nature of each variant was analyzed by nucleotide sequencing. The postion and extent of the deleted segments (shown as ---), relative to the nucleotide and amino acid sequence of pHXB2gpt, are shown diagrammatically in the middle of Figure 1. Each of the plasmid clones Δ X-A, Δ X-B, ΔX-C, Δ X-D, X9-3, X10-1 and pHXB2gpt were introduced into H9 cells using a protoplast fusioin technique; $10^{10}$ plasmid bearing bacterial protoplasts were combined with 3 x $10^6$ H9 cells, treated for one minute with 48% polyethylene glycol, diluted, washed and returned to culture in RPMI 1640 medium containing 20% fetal calf serum and antibiotics. The results shown in the lower section of Figure 1 summarize duplicate transfections performed independently. The appearance of multinucleated cells in culture is indicated + (> 1%) and was assessed by examining Wright-Giemsa stained cytocentrifuge preparations of cells removed 3 to 5 days after transfection. Expression of HTLV-III gag proteins p17 and p24 and env protein p41 was determined 2 weeks after transfection using a standard immunofluorescence approach and the monoclonal antibodies BT2, BT3 and M25, respectively. In Figure 1, a positive indicates expression by > 10% of cells; a negative indicates no expressing cells were detected. The presence (+) or absence (-) of HTLV-III virions in cultures 2 weeks after transfection was determined by electron microscopy.

Example 2

Infection of H9 cells by HTLV-III variants.
(A) Concentrated virus stocks prepared from X10-1 (■ - ■), X-C (● - ●) and pHXB2D (▲ - ▲) transfected cells were used to infect H9 cells at a multiplicity of $10^5$ particles/cell. The kinetics of cell growth of these cultures was compared with that of parallel uninfected cultures (O-O).

(B) Following exposure to virus ($10^5$ to $10^3$ virions/cell), the proportion of cells expressing HTLV-III RNA (> 20 grains/cell) expressed as a percentage of total, was determined by in situ assay using the approach of Harper et al, Proc. Natl. Acad. Sci. USA, 83:772-776 (1986) and a $^{35}$S labelled probe generated by transcription of clone pBH10-R3.

Methods. Virus stocks were prepared by harvesting 2-4 liters of the culture supernatant from exponentially growing H9 transfected cultures, centrifuging the media at 100,000 g/4° overnight and resuspending the pellet containing virus in 2-4 mls of RPMI 1640 media containing fetal calf serum and antibiotics. The concentration of virus particles per milliliter was assessed by electron microscopy and the stocks maintained under liquid nitrogen prior to use. Infection was achieved by incubating $2 \times 10^6$ prewashed H9 cells in RPMI 1640 media containing 2ug/ml of polybrene for 20 minutes at 37° C, washing the cells, resuspending the cells in 0.5 ml of media containing $2 \times 10^{11}$ or $2 \times 10^9$ virus particles and incubating for 1 hour at 37° C with gentle agitation. The cells were returned to culture (37° C, 5% $CO_2$ in a humidified atmosphere) in 5 mls of RPMI 1640 media containing 20% fetal calf serum and antibiotics and maintained at a density of $5 \times 10^5$ to $2 \times 10^6$ cells/ml for 2-4 weeks by addition of fresh media.

Example 3

Kinetics of growth and HTLV-III expression following transfection of cord blood cells with HTLV-III variant clones. Cord blood mononuclear cells were fused with bacterial protoplasts carrying the plasmids pHXB2D (O-O), pSP65gpt (□ - □), X10-1 (●-●), pHXB2-gpt (▲ - ▲), pHXB2hygro (△ - △) and △ X-C (■ - ■). The results shown in Figure 3 are the mean values obtained from three experiments performed independently. The growth pSP65gpt control cultures were comparable in experiments corresponding to Figures 3A and 3B and have hence been omitted from the latter. The percentage of cells expressing HTLV-III RNA ( >20 grains/cell) assessed by in situ assay are indicated on the right hand ordinate of Figures 3A and 3B, and the ranges of values are shown as bars.

Methods. Cord blood mononuclear cells, obtained from normal donors were separated using Ficoll-Triosil and cultured for 5 days in media containing PHA. Protoplast fusion was performed according to the approach of Fisher et al, Nature, 316:262-265 (1985), using $10^{10}$ protoplasts and $3 \times 10^6$ cells. Following transfection, the cells were maintained at $5 \times 10^5$/ml by addition of RPMI 1640 media containing 20% fetal calf serum, antibiotics and 10% interleukin-2. Cell viability was assessed by trypan blue exclusion and/or phase contrast microscopy. In situ assays were performed on cytocentrifuge preparations of cells hydridized to probe generated from pBH10-R3.

Example 4

Cytopathic effects of HTLV-III variants on ATH8 cells. Virus stocks prepared from X10-1, △ X-C, pHXB2D (cultures A and B) transfected H9 cultures or virus generated from the H9/HTLV-III$_B$ cell line were used to infect ATH8 cells at the following multiplicities of infection; $10^4$ virions/cell (●-●), $3 \times 10^3$ virions/cell (▲ - ▲) and 500 virions/cell (▽ - ▽). Control cultures, similarly treated but not exposed to virus (O-O), were also established.

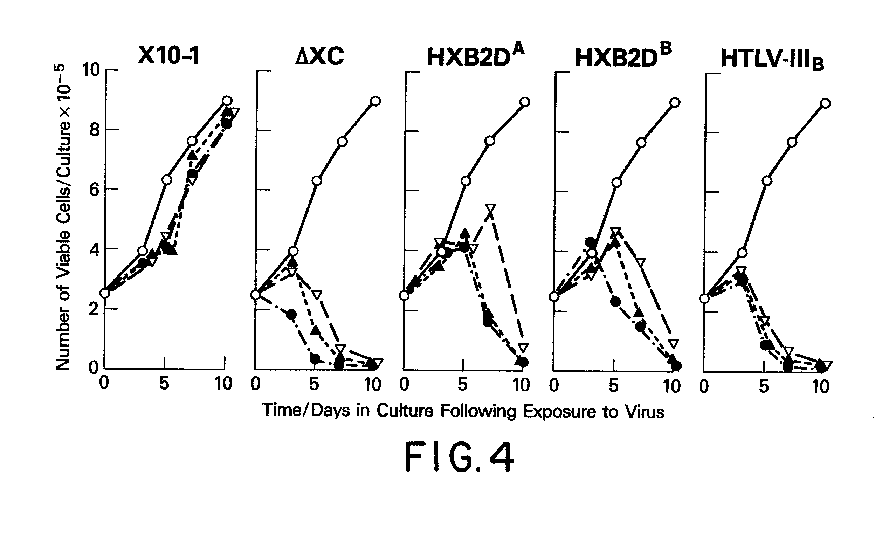

Method. Virus stocks were prepared as described in Example 2. $2.5 \times 10^5$ ATH8 cells were pretreated with polybrene (2ug/ml for 30 minutes at 37° C), exposed to virus for 45 minutes, washed and cultured in tubes containing RPMI 1640 media with 15% fetal calf serum, 15% interleukin-2 and antibiotics. The total number of viable cells was assessed using the trypan blue exclusion method. Results are shown in Figure 4.

Example 5

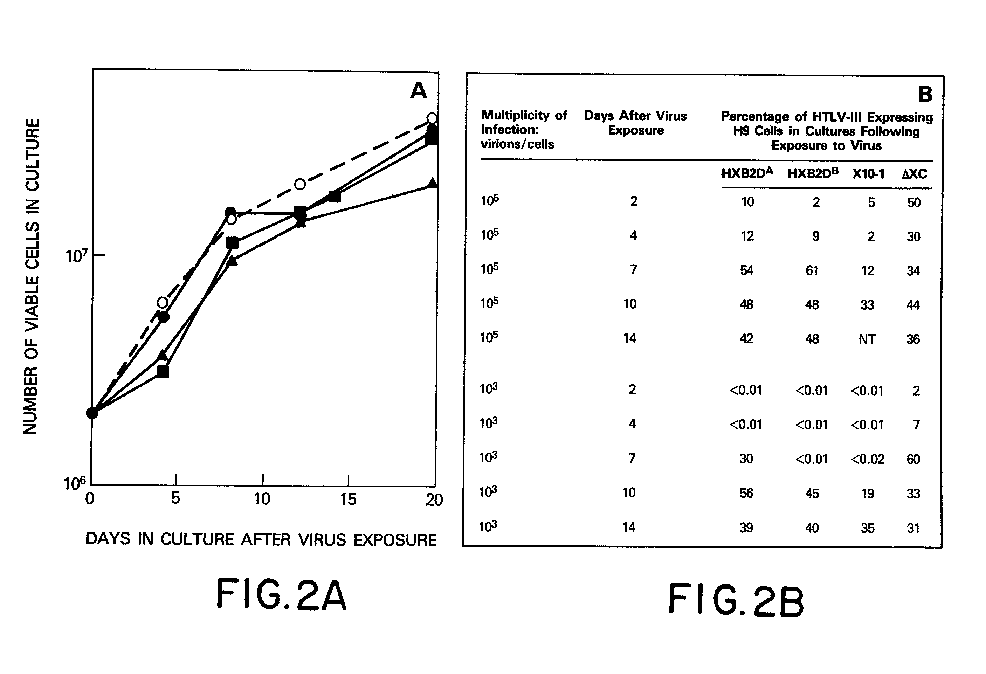

The infectivity and cytopathic potential of two HTLV-III deletion clones,△ X-C and X10-1, were studied in detail. Concentrated virus stocks were prepared by direct centrifugation of supernatants harvested 6 to 8 weeks after transfection of H9 cells with either pHXB2D, △ X-C or X10-1. These virus preparations were used to infect "virgin" H9 cultures (cells which are susceptible to productive HTLV-III infection without succumbing to the cytopathic effect of the virus). As shown in Figure 2A, the growth of H9 cell cultures was not significantly diminished following infection with large numbers ($10^5$ virions/cell) of pHXB2D, △ X-C or X10-1 derived virus. The proportion of infected cells in the cultures was estimated as the percentage of cells expressing HTLV-III RNA, detected by in situ hybridization (see Figure 2B). Virus generated from △ X-C was highly infectious, yielding 30-50% HTLV-III positive cells in cultures following the introduction of $10^5$ particles per cell (days 2 through 14). It is possible that initial high values include passively adsorbed virus at the cell surface and hence over-estimate the number of infected cells. However, the increased incidence of HTLV-III expressing cells seen 2 to 7 days after infection with $10^3$ X-C virions/cell (2% to 60%) demonstrates the infectivity of the △X-C genome. Likewise, the proportion of HTLV-III expressing cells following X10-1 virus introduction increased over a 12-day period from 2 to 48% ($10^5$ virions/cell) and 0.01% to 40% ($10^3$ virions/cell), similar to that seen in control cultures (HXB2D$^A$ and HXB2D$^B$). These results show that the infectivity of the HTLV-III genome for H9 cells, as well as its replication competence, are not significantly compromised by the deletions in clones △ X-C and X10-1.

Example 6

To investigate the possible role of 3'orf in the cytopathic potential of HTLV-III, clones △ X-C and X10-1, together with appropriate control plasmids, were introduced into PHA stimulated normal cord blood mononuclear cell cultures using the protoplast fusion technique. As shown in Figures 3A and 3B, cultures transfected with full length HTLV-III genomes (pHXB2D, pHXB2-gpt and pHXB2hygro) grew well for 10-12 days and then abruptly declined as compared with cultures transfected with no HTLV-III sequences (pSP65gpt). These results confirm that the genome carried by pHXB2D is cytopathic for normal T cells when introduced along, linked to the guanine xanthine phosphoribosyl transferase gene (gpt) or a gene conferring resistance to hygromycin (hygro). Transfection of the deletion clone $\Delta$ X-C resulted in cell and virus growth kinetics which were indistinguishable from those obtained with the full length genomes, falling within the range obtained for pHXB2gpt and pHXB2hygro (Figure 3B). This is consistent with the $\Delta$ X-C genome being fully cytopathic for normal T cells. In contrast, the deletion clone X10-1 was non-cytopathic; the kinetics of cell growth paralleled those of cultures transfected with control plasmid pSP65gpt (Figure 3A). Infected cells, detected by in situ assay for HTLV-III RNA, were evident in X10-1 transfected cultures (4.2%, day 10) showing that this discrepancy is not the result of a failure of X10-1 to generate HTLV-III expressing cells. The proportion of HTLV-III positive cells in these cultures was, on average, slightly less than in pHXB2D transfected control cultures (7.2%, day 10).

Studies using concentrated virus stocks and an OKT4+ immortalized cell line, ATH8, were performed to further test the cytopathic potential of $\Delta$ X-C and X10-1 genomes. The cell line ATH8, originally derived from a cloned tetanus toxoid specific T cell infected with HTLV-I, was used here as an indicator due to its extreme sensitivity to killing by HTLV-III. As shown in Figure 4, using multiplicities of infection of 500, $3 \times 10^3$ and $10^4$ virions/cell, virus from the deleted clone$\Delta$ X-C, the parental clone pHXB2D and from the original H9/HTLV-III$_B$line ("polyclonal" virus) were shown to be extremely cytopathic for ATH8 cells, killing the cultures in 10 days. In contrast, virus generated from the X10-1 genome did not kill or markedly suppress the growth of ATH8 cultures even when introduced at a high multiplicity ($10^4$ virions/cell). Ten days after infection 73%, 42% and 2% of cells were HTLV-III p17 positive (assessed by immunofluorescence using BT2 antibody) in cultures infected with $10^4$, $3 \times 10^3$ and 500 X10-1 virions/cell, respectively. These results confirm the potent cytopathic nature of virus generated from $\Delta$ X-C and the capacity of X10-1 virus to infect but not kill ATH8 cells.

## Claims

1. A molecular clone of Human T-cell Lymphotropic Virus Type III, characterized in that said clone produces an infectious virus which is noncytopathic toward human T cells.

2. A molecular clone of claim 1, further characterized in that it has the characteristics of the molecular clone deposited in the American Type Culture Collection under Accession #67083.

3. A molecular clone of Human T-cell Lymphotropic Virus Type III, characterized in that it comprises a biologically active molecular clone of Human T-cell Lymphotropic Virus Type III in which about the last five codons of the envelope gene and the first 60 codons of the 3'orf gene are deleted.

4. The molecular clone of claim 3, further characterized in that a segment comprising the last 14 base pairs of the envelope gene, the 4 base pair intervening sequence between the envelope gene and the 3'orf gene, and the first 182 base pairs of the 3'orf gene are deleted from said molecular clone.

5. A process for producing a noncytopathic variant of Human T-cell Lymphotropic Virus Type III, characterized by deleting a segment from Human T-cell Lymphotropic Virus Type III, said segment comprising about 65 codons of the 3'orf gene and the envelope gene.

6. The process of claim 5, further characterized in that said segment comprises the last 14 base pairs of the envelope gene and the first 182 base pairs of the 3'orf gene.

7. A molecular clone of Human T-cell Lymphotropic Virus Type III, characterized in that it comprises a biologically active molecular clone of Human T-cell Lymphotropic Virus Type III in which a segment including the last five codons of the envelope gene is deleted.

8. The molecular clone of Claim 7, further characterized in that said segment further includes 25 codons of the 3'orf gene.

9. A molecular clone, characterized in that it is the product of the process of claim 5.

10. A molecular clone, characterized in that it is the product of the process of claim 6.

11. A medicament for use in the treatment or prevention of AIDS characterised by comprising a molecular clone as claimed in any of claims 1-4 and 7-10.

12. The use of a molecular clone as claimed in any of claims 1-4 and 7-10 in the manufacture of a medicament for the treatment or prevention of AIDS.

FIG.1

FIG.2A

| Multiplicity of Infection: virions/cells | Days After Virus Exposure | Percentage of HTLV-III Expressing H9 Cells in Cultures Following Exposure to Virus | | | |
|---|---|---|---|---|---|
| | | HXB2D[A] | HXB2D[B] | X10-1 | ΔXC |
| $10^5$ | 2 | 10 | 2 | 5 | 50 |
| $10^5$ | 4 | 12 | 9 | 2 | 30 |
| $10^5$ | 7 | 54 | 61 | 12 | 34 |
| $10^5$ | 10 | 48 | 48 | 33 | 44 |
| $10^5$ | 14 | 42 | 48 | NT | 36 |
| $10^3$ | 2 | <0.01 | <0.01 | <0.01 | 2 |
| $10^3$ | 4 | <0.01 | <0.01 | <0.01 | 7 |
| $10^3$ | 7 | 30 | <0.01 | <0.02 | 60 |
| $10^3$ | 10 | 56 | 45 | 19 | 33 |
| $10^3$ | 14 | 39 | 40 | 35 | 31 |

FIG.2B

0242216

FIG.3A

FIG.3B

FIG.4

X10-1   ΔXC   HXB2D^A   HXB2D^B   HTLV-III_B

Number of Viable Cells/Culture × $10^{-5}$

Time/Days in Culture Following Exposure to Virus

0242216

| | DOCUMENTS CONSIDERED TO BE RELEVANT | | EP 87303363.3 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | NATURE, vol. 316, July 18, 1985 (New York, London) <br><br> A.G. FISHER et al. "A molecular clone of HTLV-III with biological activity" <br> pages 262-265 <br><br> * Totality * <br><br> -- | 1,7 | C 12 N 15/00 <br> C 12 N  7/00 <br> A 61 K 39/12 |
| A | EP - A1 - 0 173 529 (THE UNITED STATES OF AMERICA AS REPRESENTED BY THE SECRETARY UNITED STATES DEPARTMENT OF COMMERCE) <br><br> * Abstract; claims 1,13,14 * <br><br> -- | 1,7, 11,12 | |
| A | NATURE, vol. 312, November 8, 1984 (New York, London) <br><br> B.H. HAHN et al. "Molecular cloning and characterization of the HTLV-III virus associated with AIDS" <br> pages 166-169 <br><br> * Totality * <br><br> -- | 1,7 | **TECHNICAL FIELDS SEARCHED (Int. Cl.4)** <br><br> C 12 N <br> A 61 K |
| D,A | SCIENCE, vol. 226, no. 4679, December 7, 1984 (Washington, D.C.) <br><br> G.M. SHAW et al. "Molecular Characterization of Human T-Cell Leukemia (Lymphotropic) Virus Type III in the Acquired Immune Deficieny Syndrome" <br> pages 1165-1171 <br><br> * Totality * <br><br> -- | 1 | |

The present search report has been drawn up for all claims

| Place of search | Date of completion of the search | Examiner |
|---|---|---|
| VIENNA | 27-07-1987 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

**EUROPEAN SEARCH REPORT**

. Application number

| | DOCUMENTS CONSIDERED TO BE RELEVANT | | EP 87303363.3 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | NATURE, vol. 313, January 24, 1985 (New York, London)<br>L. RATNER et al. "Complete nucleotide sequence of the AIDS virus, HTLV-III"<br>pages 277-284<br>   * Totality *<br><br>---- | 1 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 27-07-1987 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document